(19) Europäisches Patentamt
European Patent Office
Office européen des brevets

(11) Publication number: 0 259 838 A2

(12) **EUROPEAN PATENT APPLICATION**

(21) Application number: 87113121.5

(22) Date of filing: 08.09.87

(51) Int. Cl.4: A61K 31/47 ,
//(A61K31/47,31:44)

(30) Priority: 09.09.86 US 905361

(43) Date of publication of application:
16.03.88 Bulletin 88/11

(84) Designated Contracting States:
AT BE CH DE ES FR GB GR IT LI LU NL SE

(71) Applicant: SYNTEX (U.S.A.) INC.
3401 Hillview Avenue
Palo Alto, California 94304(US)

(72) Inventor: Strosberg, Arthur M.
120 Lucero Way
Portola Valley California 94025(US)
Inventor: Whiting, Roger L.
1848 Fallenleaf Lane
Los Altos California 94022(US)

(74) Representative: Barz, Peter, Dr. et al
Patentanwälte Dipl.-Ing. G. Dannenberg Dr.
P. Weinhold, Dr. D. Gudel Dipl.-Ing. S.
Schubert, Dr. P. Barz Siegfriedstrasse 8
D-8000 München 40(DE)

(54) **Pharmaceutical composition containing A 1,4-dihydropyridine derivative and an acylated 1,2,3,4-tetrahydroisoquinoline-3-carboxylic acid.**

(57) Pharmaceutical compositions comprising a 5-(mono-or di-substituted amino) alkyl ester 1,4-dihydropyridine derivative or a pharmaceutically acceptable salt thereof and 2-{2-[(1-ethoxycarbonyl)-3-phenylpropyl]amino}-1-oxopropyl-1,2,3,4-tetrahydro-6,7-dimethoxy-3-isoquinoline carboxylic acid or a lower alkyl acid ester, or the corresponding diacid, or the pharmaceutically acceptable salts thereof, and their use in treating diseases which are affected by calcium entry blockade asnd angiotensin converting enzyme inhibition.

EP 0 259 838 A2

# PHARMACEUTICAL COMPOSITION CONTAINING A 1,4-DIHYDROPYRIDINE DERIVATIVE AND AN ACYLATED 1,2,3,4-TETRAHYDROISOQUINOLINE-3-CARBOXYLIC ACID

This invention is concerned with compositions and methods useful for treating diseases in mammals, which diseases are affected by calcium entry blockade and angiotensin converting enzyme inhibition. In particular, the invention relates to pharmaceutical compositions comprising a 1,4-dihydropyridine derivative or a pharmaceutically acceptable salt thereof and an acylated 1,2,3,4-tetrahydroisoquinoline-3-carboxylic acid or a lower alkyl acid ester, or the corresponding diacid, or the pharmaceutically acceptable salts thereof, and their use in treating cardiovascular and cerebrovascular disorders.

The present day treatment of cardiovascular and cerebrovascular disorders, including angina, hypertension, myocardial and peripheral vascular disease, congestive heart failure, ischemia, subarachnoid hemorrhage and many other conditions involving the cardiovascular and cerebrovascular systems, relies primarily on pharmacological intervention which causes peripheral and cerebral vascular dilution and a lowering of blood pressure. Only a few classes of pharmacologic agents are known to be effective; these include the alpha and beta receptor blocking agents, inotropic agents, calcium entry blocking agents and angiotensin converting enzyme (ACE) inhibitors. Within these classes, a variety of drugs have been developed which operate in the cardiovascular and cerebrovascular systems to produce somewhat different pharmacologic activity and side effect profiles.

However, no single representative or combination of any of these compounds is regarded as ideal. A novel combination of two types of cardiovascular and cerebrovascular agents has now been discovered which provides the most beneficial properties and synergistic action of both agents, and surprisingly, demonstrates a lowered incidence and degree of unwanted side effects than results from administration of members of either class of compounds individually.

The calcium entry blockers of interest in this invention are a particular class of 1,4-dihydropyridines having a broad spectrum of cardiovascular and cerebrovascular activities including anti-anginal, antihypertensive and anti-ischemic properties. The compounds of interest have a mono-or di-substituted aminoalkylcarboxy moiety at the 5-position of the pyridine ring. These compounds possess a particularly advantageous pharmacological profile, in that they possess potent cerebral and peripheral vascular dilatory activity, and in addition provide cell protection from ischemic injury. However, like all known 1,4-dihydropyridines, these compounds demonstrate some undesirable dose-related vasodilatory effects at therapeutic doses, including pedal edema, tachycardia, headache and flushing. While the flushing, edema and tachycardia seen with the calcium entry blockers of interest in this invention may be more transitory and of lesser magnitude than that seen with other dihydropyridines, they are nonetheless problematic for some patients.

Of particular interest is 2,6-dimethyl-4-(3′-nitrophenyl)-1,4-dihydropyridine-3,5-dicarboxylic acid-3-methyl ester-5-($\beta$-N-benzyl-N-methylamino)-ethyl ester, i.e. nicardipine, and its pharmaceutically acceptable salts, in particular its hydrochloride salt. Methods of making and using this and other structurally related 1,4-dihydropyridine-3,5-dicarboxylic acid alkyl esters are taught in U.S. Pat. No. 3,985,758.

Blood pressure can also be lowered by angiotensin converting enzyme (ACE) inhibitors. The particular ACE inhibitors utilized in the present invention are 2-{2-[(1-ethoxycarbonyl)-3-phenylpropyl]amino}-1-oxopropyl-1,2,3,4-tetrahydro-6,7-dimethoxy-3-isoquinoline carboxylic acid, its lower alkyl acid esters, its corresponding diacid, and its pharmaceutically acceptable salts. Particularly preferred is the hydrochloride salt of 2-{2-[(1-ethoxycarbonyl)-3-phenylpropyl]amino}-1-oxopropyl-1,2,3,4-tetrahydro-6,7-dimethoxy-3-isoquinoline carboxylic acid. Methods of making and using these compounds, and a description of their properties are disclosed in U.S. Pat. No. 4,344,949.

The ACE inhibitors used in this invention provide inhibition of the biosynthesis of angiotensin II, decreased release of aldosterone, decreased sodium retention, augmentation of the vasodilatory activity of bradykinin and subsequent vasodilation. Due to their vasodilatory activities, they are useful in the treatment of hypertension, congestive heart failure, angina and potentially in peripheral and cerebrovascular disease.

However, occasional rashes and taste disturbances, as well as rarer indidences of hematological disturbances, severe hypotension, acute renal failure and angioneurotic edema have been observed in some patients treated with ACE inhibitors. The incidence rate and severity of these effects may be lowered by the combined adminstration of the ACE inhibitor of Formula 2 below with a calcium entry blocker of Formula 1. McGregor et al, J. Cardiovasc. Pharmacol., 7 (Supp. 1) 1985, p. 52, discusses some of the effects of aCE inhibitors in combination with conventional anti-hypertensives, including the calcium channel blocker nifedipine for the treatment of severe hypertension.

European Patent Application Number 85112661.5 (Publication Number 180785) discloses a combination of 1,4-dihydropyridines with ACE inhibitors, in particular the combination of intrendipine and enalapril. The patent application inter alia also discusses the possibility of administering a combination of nisoldipine, nicardipine or felodipine with enalapril or lisinopril. However, the patent application does not disclose the combination of any 1,4-dihydropyridines with ACE inhibitors derived from any cyclic imino acids other than proline, thiaproline, 4-methoxy-proline or 3,4-dihydroproline. Specifically, the reference does not disclose ACE inhibitors derived from bicyclic imino acids as possible compounds of a combination with nicardipine.

The present invention relates to a pharmaceutical composition for treating mammalian diseases which are affected by calcium entry blockade and angiotensin converting enzyme inhibition, which composition comprises a therapeutically effective amount of the combination of a compound of Formula 1, having the formula:

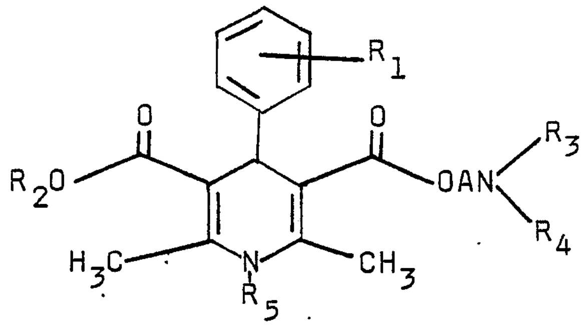

(1)

in which: $R_1$ is ortho or meta substituted $-NO_2-$, $-CF_3-$, or halo;
$R_2$ is lower alkyl or $-CH_2CH_2OCH_3$;
A is lower alkylene;
$R_3$ is lower alkyl, lower alkoxy, or optionally substituted phenyl or phenyl lower alkyl;
$R_4$ is hydrogen or lower alkyl; and
$R_5$ is hydrogen or lower alkyl;
or a pharmaceutically acceptable salt thereof, with a compound of Formula 2, having the formula:

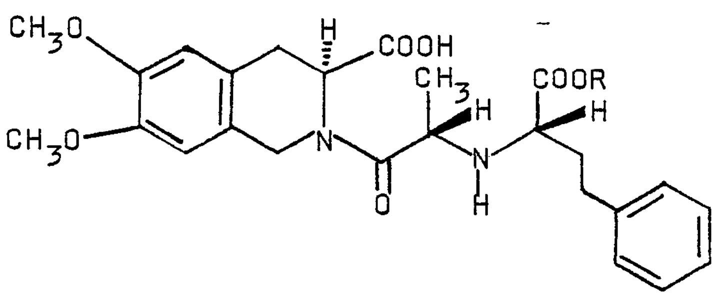

(2)

in which R is hydrogen or lower alkyl having one to four carbon atoms,
or a pharmaceutically acceptable salt thereof.

Another aspect of this invention provides a pharmaceutical composition comprising a therapeutically effective amount of a combination of a compound of Formula 1 or a pharmaceutically acceptable salt thereof with a compound of Formula 2 or a pharmaceutically acceptable salt thereof, in admixture with one or more pharmaceutically acceptable excipients.

Yet another aspect of the invention is a method of treating mammalian diseases which are affected by calcium entry blockade and angiotensin converting enzyme inhibition by administering the compounds of Formulas 1 and 2 or their pharmaceutically acceptable salts thereof in a combined amount which is therapeutically effective.

A further aspect of the invention is a method of treating mammalian diseases which are affected by calcium entry blockade and angiotensin converting enzyme inhibition, which method comprises administering a pharmaceutical composition containing a therapeutically effective amount of a combination of a compound of Formula 1 or a pharmaceutically acceptable salt thereof with a compound of Formula 2 or a pharmaceutically acceptable salt thereof.

Still another aspect of the invention is a method of preparing pharmaceutical compositions of this invention by combining the compounds of Formulas 1 and 2 or their pharmaceutically acceptable salts thereof, and optionally one or more pharmaceutically acceptable excipients, in a pharmaceutically useful dosage form and the use of the two types of compounds to prepare such dosage forms.

The combination of a compound of Formula 1 with a compound of Formula 2 provides a pharmaceutical composition which possesses unique and synergistic effect and therapeutically advantageous pharmacologic and clinical properties. In particular, this novel composition provides potent, progressive and long lasting antihypertensive effects, and also protects cells from the injury of ischemia.

Surprisingly, these compositions show significantly less of the undesirable vasodilatory effects normally associated with 1,4-dihydropyridines, such as flushing, headache, tachycardia and edema at therapeutically effective levels.

A further advantage of the invention relates to dosing and administration. While the compounds of Formula 1 are normally required to be administered at least three times daily at levels of 20 to 40 mg per dose when administered orally, the pharmaceutical composition of this invention provides therapeutic efficacy with only once or twice daily oral administration and substantially lowered effective dialy doses of both drug components. This particular aspect of the invention provides the additional advantage of improving patient compliance with the prescribed oral dosage regimen.

## Definitions

Unless otherwise stated, the following definitions apply to the defined terms as they appear in the specification and appended claims.

The term "lower alkyl" refers to saturated branched and straight chain hydrocarbon radicals having one to four carbon atoms. Typical alkyl groups include methyl, ethyl, n-propyl, i-propyl, n-butyl, i-butyl, s-butyl, t-butyl and the like.

The term "lower alkylene" refers to a branched or straight chain hydrocarbon bridging group having one to four carbon atoms, including but not limited to, methylene, ethylene, isopropylene, n-propylene, n-butylene, sec-butylene, isobutylene and the like.

The term "optionally substituted phenyl or phenyl lower alkyl" means that the phenyl group may bear a halo or lower alkoxy substituent. Examples of such groups are p-methoxybenzyl, p-chlorobenzyl, o-chlorophenyl, o-bromophenyl, m-methoxyphenyl and the like.

The term "halo" means fluoro, chloro, bromo and iodo.

The term "lower alkoxy" means the group -O-lower alkyl in which lower alkyl has the above definition.

The term "pharmaceutically acceptable salt(s)" refers to salts of the subject compounds which possess the desired pharmacological activity and which are neither biologically nor otherwise undesirable. Preferred are the acid addition salts formed by conventional reactions by treating with a stoichiometric excess of the appropriate acid. Typically, the free base is disssolved in a solvent in which the salt is insoluble. Sutiable salts include but are not limited to those formed with inorganic acids such as hydrochloric acid, hydrobromic acid, sulfuric acid, nitric acid or phosphoric acid; or with organic acids such as acetic acid, propionic acid, glycolic acid, pyruvic acid, malonic acid, succinic acid, malic acid, maleic acid, furmaric acid, tartaric acid, citric acid, benzoic acid, cinnamic acid, mandelic acid, methanesulfonic acid, ethanesulfonic acid, p-toluenesulfonic acid and the like.

The terms "optional" and "optionally" mean that the subsequently described event or circumstance may or may not occur, and that the description includes instances where said event or circumstance occurs and instances where it does not.

The term "treatment" as used herein refers to any treatment of a disease in a mammal, paricularly a human, and includes:

(i) preventing the diseases from occurring in a subject which may be predisposed to the disease but has not yet been diagnosed as having it;

(ii) inhibiting the disease, i.e., arresting its development; or

(iii) relieving the disease, i.e., causing regression of the disease.

The term "disease" refers to any physiological state which is capable of being affected in a beneficial manner by administration of a therapeutically active agent.

The compounds of Formula 2 have three asymmetric carbon atoms. The compounds used in this invention have the L(S) configuration. This configuration has been shown to be required for biological activity, and thus active compounds of Formula 2 are derived from either (L-) or DL-1,2,3,3-tetrahydroisoquinoline-3-carboxylic acids. The preferred compounds for use in this invention are those having the S configuration at each of the three chiral centers. However, the various optical and diastereo isomers arising from the chirality of the three centers of the compounds of Formula 2 are within the scope of this invention to the extent such isomers possess ACE inhibitory activity.

Phrases such as "compound of Formula 1" and "compound of Formula 2" refer to the generic structures of the compounds of the invention composition. The generic structure depicted for compounds of Formula 2 indicates the preferred S,S,S, stereochemistry of the compounds. However, other isomers are included within this invention, to the extent stated above.

Preferred pharmaceutical compositions are derived from compounds of the formula (1) wherein $R_1$ is $-NO_2$, $R_2$ is lower alkyl, in particular methyl, $R_3$ is phenyl lower alkyl, in particular benzyl, $R_4$ is lower alkyl, in particular methyl, $R_5$ is hydrogen and A is methylene to n-propylene, in particular ethylene.

The most preferred pharmaceutical compositions of this invention comprise a combination of nicardipine (2,6-dimethyl-4-(3′-nitrophenyl)-1,4-dihydropyridine-3,5-dicarboxylic acid-3-methyl ester-5-β-(N-benzyl-N-methylamino)-ethyl ester), or a pharmaceutically acceptable salt thereof, and 2-{2-[(1-ethoxycarbonyl)-3-phenylpropyl]amino}-1-oxopropyl-1,2,3,4-tetrahydro-6,7-dimethoxy-3-isoquinoline carboxylic acid, or a pharmaceutically acceptable salt thereof. The preferred forms of nicardipine and 2-{2-[(1-ethoxycarbonyl)-3-phenylpropyl]amino}-1-oxopropyl-1,2,3,4-tetrahydro-6,7-dimethoxy-3-isoquinoline carboxylic acid are as the hydrochloride salts. Another preferred compound of Formula 2 is 2-{2-[(1-methoxycarbonyl)-3-phenylpropyl]amino}-1-oxopropyl-1,2,3,4-tetrahydro-6,7-dimethoxy-3-isoquinoline carboxylic acid and the pharmaceutically acceptable salt thereof, preferably the acid addition salts.

The presently preferred embodiments also include:

a pharmaceutical composition useful for treating one or more cardiovascular diseases, such as hypertension, angina, myocardial infarction, as well as cerebral and peripheral vascular diseases, in a mammal, particularly a human being, which composition comprises a therapeutically effective amount of a combination of a compound of Formula 1 and a compound of Formula 2, or pharmaceutically acceptable salts thereof, optionally in admixture with a pharmaceutically acceptable excipient; and

a method for treating one or more cardiovascular, cerebrovascular and peripheral vascular diseases in a mammal, particular in a human being, which comprises administering to a subject in need of such treatment a therapeutically effective amount of a combination of a compound of Formula 1 with a compound of Formula 2, or pharmaceutically acceptable salts thereof.

## UTILITY AND ADMINISTRATION

The pharmaceutical compositions of the present invention comprise the combination of a calcium channel blocker with an ACE (angiotensin converting enzyme) inhibitor. As such, they possess all of the beneficial cardiovascular pharmacologic properties properties of both component molecules.

The compounds of Formula 1 inhibit calcium ion influx through slow channels into smooth muscle, cardiac and neural cells. As a result, they possess potent peripheral and cerebral vasodilatory activity and cause a decrease in blood pressure. In addition, they provide cell protection to tissue subjected to ischemic insult, both before and after the onset of an ischemic incident. Therefore, they are particularly useful for treating myocardial, peripheral and cerebral vascular disorders.

The ACE inhibitors of Formula 2 block the conversion of the decapeptide angiotensin I to angiotensin II, and thus lower blood pressure by inhibiting the biosynthesis of angiotensin II in the periphery, especially in animals and humans whose hypertension is angiotensin II related. In addition, compounds of Formula 2 may inhibit the biosynthesis of angiotensin II in the central nervous system. ACE degrades the vasodilating substance bradykinin. Therefore, ACE inhibitors may lower blood pressure by potentiating bradyinin's effects, as well as by inhibiting the biosynthesis of angiotensin II. Although the relative importance of these and other possible mechanisms remains to be established, ACE inhibitors are known to be effective vasodilatory agents in a variety of animal models and are useful clinically, for example, in many human patients with malignant renovascular and essential hypertension, and congestive heart failure.

A number of in vitro and in vivo models are available to assess the biological acitivity of ACE inhibitors. (cf E. W. Petrillo, Jr. and M A. Ondetti, Medical Res. Rev . 2, 1 (1982) and references therein). A particularly useful in vitro test employs a preparation of ACE from rabbit lung (either crude or purified) and uses the synthetic substrate (hippuryl-histidyl-leucine) as substrate (D. W. Cushman and H. S. Cheung, Biochem.

Pharmacol., 20, 1637 (1971)). The substances to be tested are assessed for their ability to block the cleavage of the substrate, and doses which cause 50% inhibition of the hydrolysis of the substrate are determined ($ID_{50}$). In this and other studies, the incorporation of a literature standard (usually captopril) is helpful.

A useful in vivo model uses the normotensive rat which is challenged with doses of angiotensin I. Administration of the test drug, either I.V. or by the oral route (p.o) results in a dose related suppression of the hypertensive response to the angiotensin I challenge, either I.V. or intracerebroventricularly (I.C.V.). (D. M. Gross, et al, J. Pharmacol. Exp. Ther.,216, 522 (1981).

The compounds of Formula 2 disclosed herein have proven to be potent and long-acting ACE inhibitors both in vitro and in vivo.

As previously discussed, the novel pharmaceutical compositions of this invention provide the beneficial therapeutic properties of both drug components, including all of the pharmacologic modes of action described above for the compounds of Formulas 1 and 2. Thus, these compositions provide potent, progressive and long lasting antihypertensive effects, and also protect cells from injury from ischemia. Additionally, these compositions show significantly less of the undesirable vasodilatory side effects of compounds of Formula 1 such as flushing, headache, tachycardia and edema at therapeutically effective levels. These vasodilatory side effects, particularly flushing, can be measured in animal models. For example, in the four vessel occlusion stroke model (B.J. Alps and W. Hass, Neurology 35, (Suppl. 1), 141, 1985), flushing can be detected by redness of the ears. Therefore, an equivalent effect of compounds of Formula 1 and of the combination of this invention on stroke can be produced, and the relative amounts of resultant flushing discriminated. Another useful laboratory model for determining vasodilatory side effects is the spontaneously hypertensive rat. In this model, dose-related decreases in blood pressure and accompanying dose-related increases in tachycardia and flushing are monitored following administration of the test compound or composition. In this way, comparisons can be made of the incidence and degree of tachycardia and flushing resulting from administration of compounds and compositions at levels which result in equivalent blood pressue lowering.

Thus, the compositions of this invention are useful in treating cardiovascular and cerebrovascular disorders in patients who are susceptible to calcium ion entry blockade and angiotensin converting enzyme inhibition. These cardiovascular and cerebrovascular disorders may include various forms of hypertension, angina, heart failure, peripheral and cerebral vascular diseases, cardiac insufficiency and coronary heart diease.

Administration of the pharmaceutical compositions of this invention can be by any of the normally accepted modes of administration for therapeutic agents. These methods include oral, parenteral, transdermal, subcutaneous an other systemic modes. The preferred method of administration is oral, except in those cases where the subject is unable to ingest, by himself, any medication. In those instances, it may be necessary to administer the composition parenterally.

Depending on the intended mode of administration, the compositions used may be in the form of solid, semi-solid or liquid dosage forms, such as, for example, tablets, suppositories, pills, capsules, powders, liquids, suspensions, or the like, preferably in unit dosage forms suitable for single administration of precise dosages. The compounds of Formulas 1 and 2 or their pharmaceutically acceptable salts thereof may be administered in combination in a single composition, or may be administered in individual compositions. Administration of the compounds of Formulas 1 and 2 or their pharmaceutically acceptable salts thereof in a single composition is preferred, since it facilitates administration and minimizes the opportunities for inaccurate dosing. In any event, the compositions may include a conventional pharmaceutical carrier or excipient in admixture with an active compound of Formula 1 or a pharmaceutically acceptable salt thereof, or an active compound of Formula 2 or a pharmaceutically acceptable salt thereof, or active compound or salt of Formula 1 and Formula 2. In addition, the compositions may include other medicinal agents, pharmaceutical agents, carriers, adjuvants, etc. which do not unduly comprise the beneficial biological and pharmacological characteristics of the composition.

For solid compositions, conventional non-toxic solid carriers include, for example, pharmaceutical grades of mannitol, lactose, starch, magnesium stearate, sodium saccharin, talcum, cellulose, glucose, sucrose, magnesium carbonate, and the like. The active compounds as defined above may be formulated in suppositories using, for example, polyalkylene glycols, for example, propylene glycol, as the carrier. Liquid pharmaceutically administerable compositions can, for example, be prepared by dissolving, dispersing, etc. the active compounds as defined above and optional pharmaceutical adjuvants in a carrier, such as, for example, water, saline solutions, aqueous dextrose, glycerol, ethanol, and the like, to thereby form a solution or suspension. If desired, the pharmaceutical, composition to be administered may also contain mirror amounts of nontoxic auxiliary substances such as wetting or emulsifying agents, pH buffering agents

and the like, for example, sodium acetate, sorbitan monolaurate, triethanolamine sodium acetate, triethanolamine oleate, etc. Actual methods of preparing such dosage forms are known, or will be apparent, to those skilled in this art; for example, see Remington's Pharmaceutical Sciences, Mack Publishing Company, Easton, Pennsylvania, 16th Edition, 1980. The composition or formulation to be administered will, in any event, contain a quantity of the active compounds of Formula 1 and 2 in an amount effective to alleviate the symptoms of the subject being treated.

For oral administration, a pharmaceutically acceptable non-toxic composition may be formed by the incorporation of any of the normally employed excipients, such as, for example, pharmaceutical grades of mannitol, lactose, starch, magnesium stearate, sodium saccharin, talcum, cellulose, glucose, sucrose, magnesium carbonate, and the like. Such compositions take the form of solutions, suspensions, tablets, pills, capsules, powders, sustained release formulations and the like. Such compositions may contain 1%-95% active ingredients, preferably 25-70%.

The amount of each active compound being administered in the pharmaceutical composition will depend upon the targeted disease state(s), as well as the subject being treated. When administered in the composition of this invention, an effective dose of the compound of Formula 1 will be in the range of 10 to 160, preferably 40 to 80, mg per patient per day, and an effective dose of the compound of Formula 2 will be in the range of 15 to 120, preferably 30 to 60, mg per patient per day. The composition will preferably contain the compounds of Formula 1 and 2 or their pharmaceutically acceptable salts thereof in a percent by weight ratio of approximately 10:90 to 90:10, preferably 25:75 to 75:25, more preferably in a ratio between 33:67 and 67:33, and will contain a quantity of the active compounds in a therapeutically effective amount, i.e. an amount effective to provide the intended therapeutic objective. The precise amount of the composition which is administered will depend on such factors as the particular condition of the subject being treated, and the judgement of the treating physician.

The following examples illustrate the preparation of representative pharmaceutical formulations suitable for practicing the invention described and claimed herein, and should not be construed as narrowing it, or limiting its scope. In Example 1 to 6, compound 1 is 2,6-dimethyl-4-(3'-nitrophenyl)-1,4-dihydropyridine-3,5-dicarboxylic acid-3-methyl ester-5-β-(N-benzyl-N-methylamino)-ethyl ester, hydrochloride and compound 2 is 2-{2-[(1-ethoxycarbonyl)-3-phenylpropyl]amino}-1-oxopropyl-1,2,3,4-tetrahydro-6,7-dimethoxy-3-isoquinoline carboxylic acid, hydrochloride, S,S,S. However, other compounds and/or salts of Formulas 1 and 2 or their pharmaceutically acceptable salts thereof can also be used.

## EXAMPLE 1

| Ingredients | Quantity per tablet, mgs. |
|---|---|
| Compound 1 | 10 |
| Compound 2 | 20 |
| cornstarch | 20 |
| lactose, spray-dried | 153 |
| magnesium stearate | 2 |

The above ingredients are thoroughly mixed and pressed into single scored tablets for oral administration.

## EXAMPLE 2

| Ingredients | Quantity per tablet, mgs. |
|---|---|
| Compound 1 | 30 |
| Compound 2 | 30 |
| cornstarch | 23 |
| lactose, spray-dried | 148 |
| magnesium stearate | 2 |

The above ingredients are mixed and introduced into a hard-shell gelatin capsule.

## EXAMPLE 3

| Ingredients | Quantity per tablet, mgs. |
|---|---|
| Compound 1 | 20 |
| Compound 2 | 10 |
| lactose | 15 |
| cornstarch | 25 |
| magnesium stearate | 2 |

The above ingredients are mixed and introduced into a hard-sheel gelatin capsule.

## EXAMPLE 4

| Ingredients | Quantity per tablet, mgs. |
|---|---|
| Compound 1 | 10 |
| Compound 2 | 60 |
| cornstarch | 24 |
| hydrogenated vegetable oil | 5 |
| microcystalline cellulose | 47 |
| lactose | 92 |

The above ingredients are mixed and introduced into a hard-shell gelatin capsule.

## EXAMPLE 5

An injectable preparation buffered to a pH of 7 is prepared having the following composition:

| Ingredients | |
|---|---|
| Compound 1 | 0.2 g |
| Compound 2 | 1.0 g |
| Acetate buffer (0.1 N solution) | 2 ml |
| KOH (1 N) | q.s. to pH 4.5-5 |
| water (distilled, sterile) | q.s. to 20 ml |

EXAMPLE 6

An oral suspension is prepared having the following composition:

Ingredients

| | |
|---|---|
| Compound 1 | 0.1 g |
| Compound 2 | 1.0 g |
| sodium chloride | 2.0 g |
| methyl paraben | 0.1 g |
| granulated sugar | 25.5 g |
| sorbitol (70% solution) | 12.85 g |
| Veegum K (Vanderbilt Co.) | 1.0 g |
| flavoring | 0.035 ml |
| colorings | 0.5 mg |
| distilled water q.s. to 100 ml | |
| fumaric acid (1% solution) | q.s. to pH 5 |

EXAMPLE 7

Method

Twenty-four renal hypertensive rates (Male Sprague-Dawley, 140-170g) showing systolic blood pressure of 160 mmHg or greater were chosen for the study. Using the random block design, they were distributed into four groups: 3 groups for compound treatments and one for vehicle control. They were randomly assigned to the treatments. The test compounds were dissolved in deionized water at concentrations such that 1.0 ml of solution was administered per 100 grams of body weight.

At approximately 17 hours prior to dosing, food was removed from the rat cages. On the morning of testing, the rats were dosed with the assigned compound or vehicle by gavage. Immediately after dosing, the rats were put in restrainers and placed in a heated chamber, 30 degrees centrigrade, for four hours. After the four hour reading, the rats were returned to their home cage for 3 hours and then were put in the restrainer again for the hour 8 reading.

Using a tail blood pressure cuff, blood pressure curves and tail pulses were simultaneously monitored on a 4-channel MFE recorder digitized by a computer system. At least four consecutive traces (at 30 second intervals) were recorded for each rat at 1, 2, 4 and 8 hour post-dose.

The computer system has been programmed to find systolic blood pressure (mmHg) and heart rate (beats/min) for each recording and them calculate the mean SBP and the mean HR of each rat at each observation time. Manual editing and confirmation of the analyses were carried out by the investigator on any systolic blood pressure traces that were more than two standard deviations outside the mean blood pressure readings for that animal at a given time period.

Statistical Analysis

The SBP's of the animals in each dose group were compared to the SBP's of the animals in the control group at each observation time using oneway analysis of variance. Significance was determined using the Dunnet test. A compound with $p<0.05$ at any observation time was considered to exhibit significant antihypertensive activity.

Results

As shown in Table 1, in the renal hypertensive rats, oral administration of both incardipine hydrochloride (3mg/kg) and 2-{2-[(1-ethoxy-carbonyl)-3-phenylpropyl]amino}-1-oxopropyl-1,2,3,4-tetrahydro-6,7-dimethoxy-3-isoquinoline carboxylic acid hydrochloride (10mg/kg) induced a greater antihypertensive effect than nicardipine (3mg/kg) or 2-{2-[(1-ethoxy-carbonyl)-3-phenylpropyl]amino}-1-oxopropyl-1,2,3,4-tetrahydro-6,7-dimethoxy-3-isoquinoline carboxylic acid (10mg/kg) alone. The potentiation effect lasted more than 4 hours.

TABLE 1

Antihypertensive Activities of Nicardipine and 2-{2-[(1-ethoxy-carbonyl)-3-phenylpropyl]amino}-1-oxopropyl-1,2,3,4-tetrahydro-6,7-dimethoxy-3-isoquinoline carboxylic acid in Renal Hypertensive Rats[a]

| Compound | Chg from Control | Hr 1 | 2 | 4 | 8 |
|---|---|---|---|---|---|
| nicardipine | SBP mmHg Chg | -5 | -7 | -10 | -35 |
| | % Chg | -3 | -4 | -6 | -20 |
| 3 mg/kg | HR b/min. Chg | 65 | 30 | 25 | -4 |
| po | % Chg | 22 | 10 | 9 | -2 |
| 2-{2-[(1-ethoxy-carbonyl)-3-phenylpropyl]-amino}-1-oxopropyl-1,2,3,4-tetrahydro-6,7-dimethoxy-3-isoquinoline carboxylic acid | SBP mmHg Chg | -14 | -13 | 4 | -12 |
| | % Chg | -8 | -7 | 3 | -7 |
| 10 mg/kg | HR b/min. Chg | 87 | 11 | 10 | -33 |
| po | % Chg | 29 | 4 | 4 | -11 |

| Compound | Chg from Control | Hr 1 | 2 | 4 | 8 |
|---|---|---|---|---|---|
| Combination nicardipine 3 mg/kg 2-{2-[(1-ethoxy-carbonyl)-3-phenylpropyl]-amino}-1-oxopropyl-1,2,3,4-tetrahydro-6,7-dimethoxy-3-isoquinoline carboxylic acid 10 mg/kg po | SBP mmHg Chg | -52* | -36* | -29 | -13 |
| | % Chg | -31 | -20 | -17 | -8 |
| | HR b/min. Chg | -19 | 46 | 19 | -6 |
| | % Chg | -7 | 15 | 7 | -2 |

[a] n = 6 for each group

* $p<0.05$, compared to control

EXAMPLE 8

Toxicology

The acute oral toxicity of concomitant administration of nicardipine hydrochloride and 2-{2-[(1-ethoxy-carbonyl)-3-phenylpropyl]-amino}-1-oxopropyl-1,2,3,4-tetrahydro-6,7-dimethoxy-3-isoquinoline carboxylic acid hydrochloride was evaluated in CD rats. Three groups, each composed of 5 male and 5 female rats were used in the study. The doses of nicardipine hydrochloride/2-{2-[(1-ethoxy-carbonyl)-3-phenylpropyl]-amino}-1-oxopropyl-1,2,3,4-tetrahydro-6,7-dimethoxy-3-isoquinoline carboxylic acid hydrochloride employed were 20/10, 50/25, and 100/50 mg/kg, respectively in the three groups. Following oral administration of single doses of each compound, the animals were observed for clinical signs of toxicity for 8 days.

Several animals from all dose groups exhibited pink skin (peripheral vasodilation) on the day of dosing. This observation was noted previously in studies in rats with nicardipine hydrochloride. The average body weight gain and food intake were slightly decreased in the high-dose group males. All animals survived the 8-day observation period.

Conculsion

Concomitant oral administration of nicardipine hydrochloride at 100mg/kg and 2-{2-[(1-ethoxy-carbonyl)-3-phenylpropyl]-amino}-1-oxopropyl-1,2,3,4-tetrahydro-6,7-dimethoxy-3-isoquinoline carboxylic acid hydrochloride at 50 mg/kg was well tolerated by the rat.

**Claims**

1. A pharmaceutical composition comprising a therapeutically effective amount of the combination of a compound of Formula 1, having the formula:

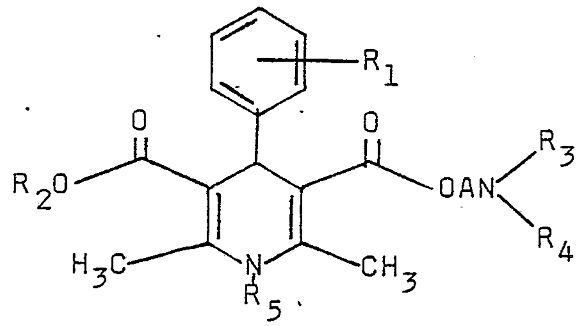

(1)

in which: $R_1$ is ortho or meta substituted $-NO_2$, $-CF_3$, or halo;
$R_2$ is lower alkyl or $-CH_2CH_2OCH_3$;
A is lower alkylene;
$R_3$ is lower alkyl, lower alkoxy, or optionally substituted phenyl or phenyl lower alkyl;
$R_4$ is hydrogen or lower alkyl; and
$R_5$ is hydrogen or lower alkyl;
or a pharmaceutically acceptable salt thereof, with a compound of Formula 2, having the formula:

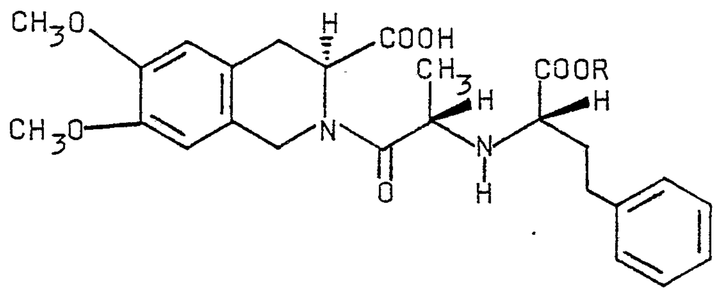

(2)

in which R is hydrogen or lower alkyl having one to four carbon atoms,
or a pharmaceutically acceptable salt thereof.

2. The pharmaceutical composition of claim 1 for the treatment of mammalian diseases which are affected by calcium entry blockage and angiotensin converting enzyme inhibition.

3. The pharmaceutical composition of claim 1 or 2 wherein $R_1$ is $-NO_2$, $R_2$ is lower alkyl, $R_3$ is phenyl lower alkyl, $R_4$ is lower alkyl, $R_5$ is hydrogen and A is methylene to n-propylene in Formula 1.

4. The pharmaceutical composition of claim 3 in which the compound of Formula 1 is 2,6-dimethyl-4-(3'-nitrophenyl)-1,4-dihydropyridine-3,5-dicarboxylic acid-3-methyl ester-5-$\beta$-(N-benzyl-N-methylamino)-ethyl ester, or a pharmaceutically acceptable salt thereof.

5. The pharmaceutical composition of any one of claims 1 to 4 in which the compound of Formula 2 is 2-{2-[(1-ethoxycarbonyl)-3-phenylpropyl]amino}-1-oxopropyl-1,2,3,4,-tetrahydro-6,7-dimethoxy-3-isoquinoline carboxylic acid, or a pharmaceutically acceptable salt thereof.

6. The pharmaceutical composition of any one of claims 1 to 4 in which the compound of Formula 2 is 2-{2-[(1-methoxycarbonyl)-3-phenylpropyl]amino}-1-oxopropyl-1,2,3,4,-tetrahydro-6,7-dimethoxy-3-isoquinoline carboxylic acid, or a pharmaceutically acceptable salt thereof.

7. The pharmaceutical composition of any one of claims 1 to 6 in which the pharmaceutically acceptable salts are the hydrochlorides.

8. The pharmaceutical composition of any one of claims 1 to 7 comprising a therapeutically effective amount of a combination of the compounds of Formulas 1 and 2 or their pharmaceutically acceptable salts , in admixture with one or more pharmaceutically acceptable excipients.

9. The pharmaceutical composition of any one of claims 1 to 8 in which the percent by weight ratio of the compound of Formula 1 to the compound of Formula 2 is between 10:90 and 90:10, preferably in a ratio between 25:75 and 75:25, more preferably in a ratio between 33:67 and 67:33.

10. The use of a combination of the compounds of Formulas 1 and 2 or their pharmaceutically acceptable salts according to any one of claims 1 to 9 in the manufacture of a medicament for treating mammalian diseases which are affected by calcium entry blockade and angiotensin converting enzyme inhibition.

11. A process for preparing a pharmaceutical composition according to ny one of claims 1 to 9 which comprises combining a compound of Formula 1 or a pharmaceutically acceptable salt thereof, preferably the hydrochloride salt, and a compound of Formula 2 or a pharmaceutically acceptable salt thereof, preferably the hydrochloride salt, in conjunction with a pharmaceutically acceptable excipient.